# EUROPEAN PATENT SPECIFICATION

(11) **EP 0 675 702 B1**
(45) Date of publication and mention of the grant of the patent: **01.04.1998**
(21) Application number: 94902929.2
(22) Date of filing: 22.12.1993
(51) Int. Cl.: A61F 13/02

(54) **ADHESIVE DRESSING**
HAFTVERBAND
PANSEMENT ADHESIF

(30) Priority: 24.12.1992 GB 9226909
(43) Date of publication of application: 11.10.1995
(73) Proprietor: Smith & Nephew plc, London WC2R 3BP (GB)
(72) Inventor: WARD, William, John, Hull HU5 3JP (GB)
(74) Representative: Koepsell, Helmut, Dipl.-Ing.
(86) International application number: GB9302623
(87) International publication number: WO9414393

(56) References cited:
- EP-A- 0 091 800
- GB-A- 2 224 445
- US-A- 4 832 008
- US-A- 5 153 040

## Description

The present invention relates to an adhesive dressing used on wound areas and in particular but not exclusively for wounds such as burns, abrasions, ulcers and surgical incisions and as a protective dressing to cover for example an indwelling catheter site and as part of an ostomy dressing for example to form the fixation for an ostomy bag.

There have been many different ways of presenting adhesive dressings, for example using a protector layer over the adhesive having adhesive free handles at a pair of opposed edges. Another mode of presentation as described in UK Patent No. 2224445 requires the presence of a carrier means in the form of a support layer usually of a material stiffer than the adhesive coated film adhered to the non-adhesive surface of the film and having a V-shaped handle formed from the protector. The support layer conforms to the contours of the skin to which it is applied and may be left attached to the dressing. However, it is preferred that it should be removed. Despite the fact that in such presentations the support layer extends beyond the adhesive coated film when the removal of the support layer is required it is often the case that by peeling back the support layer, the adhesive coated film will also be peeled away from its contact with skin. This is a distinct disadvantage and is often aggravated by the fact that in the first few seconds of contact of the adhesive surface of the film with the skin surface before the removal of the support layer, the adhesion at the edge of the adhesive coated film between the support layer and film is greater than that between the adhesive and the skin surface. The reduced adhesion between the film and the skin surface may be as a result of moisture on a skin surface or a body hair which interrupts the adhesive contact. This peel back effect may also be caused when the adhesive is pattern spread over a film, in which case there will be areas along the edge of the film which are not covered in adhesive and therefore will be liable to uplift with the support layer. The adhesive may also have low initial tack properties due to the adhesive being below skin temperature on contact with the skin and therefore does not show the same adhesive qualities as it does once the adhesive has been activated by body heat.

The present invention seeks to overcome the above-mentioned disadvantages by providing a novel dressing presentation which includes an additional component to aid the adhesion of the film to the skin.

According to one aspect of the present invention there is provided an adhesive dressing which comprises a conformable backing layer having a pressure sensitive adhesive layer over one surface thereof, a removable protector which covers the adhesive layer and extends beyond the backing layer at a contiguous edge on the backing and adhesive layers and a conformable support layer which is releasably attached to the non-adhesive surface of the backing layer and extends beyond the backing layer at said edge characterised in that attached to the adhesive layer along at least a part of said edge is a component comprising a second conformable layer having a skin contacting layer of adhesive on one surface thereof, which component extends outwardly to underlie the extended portion of the conformable support layer.

According to yet a further aspect of the present invention there is provided a method of manufacture of the dressing which comprises the steps of:-
casting a solution of a polymer which is to form a backing layer onto a strip of film forming a support layer
spreading an adhesive onto a protector layer and laminating the adhesive and film and those associated layers together,
removing the protector layer from the adhesive and, if required, trimming off a strip of film and adhesive along at least one edge of the laminate and characterised in applying a component comprising a second conformable layer, adhesive layer and protector to an edge or trimmed edge of the adhesive layer contiguous with the backing layer

Suitably the backing layer may comprise a hydrophilic polyurethane having a water uptake of 25% such as that disclosed in example 2 of United Kingdom Patent No. 2 093 190.

Alternatively the backing layer may comprise materials which are conventionally employed to form thin film surgical dressings. Suitable materials include those described in United Kingdom Patent No. 1280631, European Patents Nos.0051935, 0091800 and 0178740. Particularly apt materials are polyurethanes for example polyester or polyether polyurethanes known under the name Estane (Trade Mark) and polyether polyamides for example those known under the name Pebax (Trade Mark). Other favoured materials include hydrophilic polymers such as hydrophilic polyurethanes including those described in United Kingdom Patent No. 2093190 especially the polyurethane described in Example 2 therein. Such materials will typically take up from 5 to 95% by weight of water.

The materials employed in the dressings of the invention may be moisture vapour permeable.

The moisture vapour transmission rate of the materials employed in the present invention may be measured by a procedure known as the Payne Cup method. The method uses a cup 1.5cm deep with a flanged top. The inner diameter of the flange is such to provide an area for moisture vapour transmission of 10cm². In this method 10ml of distilled water is added to the cup and a sample of the material under test large enough to completely cover the flange, is clamped over the cup. The complete assembly is then weighed and placed in a cabinet where the temperature and relative humidity are maintained at 37°C and 10% respectively. After 17 hours the cup is removed from the cabinet and allowed to cool at room temperature. After re-weighing, the mass of water lost by vapour transmission is calculated and the result expressed as in gm-²/24hrs at 37°C at 100% to 105 relative humidity difference. Hereinafter the units for moisture vapour transmission will be abbreviated to gm⁻².

Suitably the backing layer is moisture vapour permeable and has a moisture vapour transmission rate of at least 300g m⁻²/24 hours relative humidity difference, more suitably at least 500g m⁻²/24 hours and preferably at least 800g m⁻²/24 hours.

Suitably the backing layer has thickness of from 15 to 80µm, more suitably 20 to 60µm and preferably 25 to 50µm, for example 30µm, 35µm and 40µm.

The pressure sensitive adhesive layer may preferably be formed from an adhesive which is conventionally used for contact with the skin. Most desirably, the pressure sensitive adhesive is an acrylic adhesive such as an acrylate ester copolymer adhesive formed by the copolymerisation of 2-ethyl-hexyl acrylate, butyl acrylate and acrylic acid. Alternatively, the adhesive layer may be an adhesive such as polyvinyl alkyl ether adhesive. Suitable adhesives are described in United Kingdom Patent No. 1280631 and European Patents Nos. 35399 and 51935.

The layer of adhesive provided on the backing layer may be a continuous layer of moisture vapour permeable adhesive such as acrylate surgical adhesives or polyvinyl ethyl ether. The moisture vapour permeable adhesive is preferably of a weight of 5gm⁻² to 50gm⁻² and most desirably 15gm⁻² to 25gm⁻².

Alternatively the layer of adhesive is a discontinuous layer of adhesive spread over the film in such a fashion as to allow moisture vapour transmission. This may be achieved by pattern spreading the adhesive whereby a regular repeated pattern of adhesive is spread over the film or may be achieved by spreading a porous adhesive. The porous adhesive may be microporous, or it may alternatively have macropores or a mixture of micro and macropores. The porous layer of adhesive allows access of skin surface moisture to the film. Favourably the diameter of the pores are 0.1 to 10 times the thickness of the adhesive layer. Suitably the pores will be from about 1000 µm in diameter.

Suitably the removable protector is a silicone coated release paper. Desirably the removable protector may be divided into two or more pieces. Preferably at least one of the protector pieces is significantly larger than the other or others and covers a major proportion of the adhesive layer. It is desirable that the stripping load of the support layer from the backing layer is equal to or greater than that of the protector from the adhesive layer otherwise there is a risk that the support layer would peel from the backing layer before the protector can be removed.

The removable protector most desirably covers and extends beyond the second conformable layer and skin contacting adhesives of the edge strip once it has been applied.

The support layer is aptly formed from a polymeric film or paper. The support layer may be attached to the backing layer by virtue of casting or extruding the backing layer onto the support layer thereby forming an attachment which is easily reversed. Most suitably the support layer maybe formed from for example, a transparent polymeric film such as a polyethylene or polypropylene film or from an opaque silicone or polyethylene coated paper.

The edge strip which comprises a second conformable layer having a skin contacting layer of adhesive on one surface thereof, is preferably a continuous strip attached along the edge of the backing layer. Alternatively, the edge strip may be discontinuous and be attached along only part of the edge of the backing layer. A further alternative may be the edge strip being attached along the edge of all of the backing layer.

The second conformable layer is preferably made from the same material as the backing layer and the skin contacting layer of adhesive is also preferably that used upon the backing layer. Alternatively, however, the second conformable layer may be made from polyvinyl ethylene or any of the alternative suggested for the backing layer. A further alternative may be materials including paper, non-woven fabric, woven fabric and films, sheets or webs of polymers including polypropylene, polyethylene, copolymers thereof and blends including polystyrene, polyester and polyvinyl chloride.

Particularly apt materials of the type mentioned above include paper, porous polyvinyl chloride sheet such as that sometimes known as Porvic (Trade Mark) which is conventionally used in the manufacture of first aid dressings, non-woven fabric such as spun-bonded polyester fabric (Sontara, Trade Mark), polyester film (Melinex, Trade Mark), woven acrylic fabric, embossed films of low or high density polyethylene or polypropylene, integral nets formed by the fibrillation of embossed films and oriented polypropylene films.

The adhesive dressing may be prepared by casting a solution of the polymer which is to form the backing layer onto a long strip of the film which is to form the support layer. An adhesive may then be screen printed onto the protector layer. The polymer and support layer may then be laminated to the adhesive screen printed protector layer the cast polymer to the adhesive. The laminated roll may then be trimmed to the correct laminate width. The protector layer is then peeled off the adhesive, the polymerised adhesive trimmed to the final dressing width, an edge strip and a V-handle placed in position and then the protector layer replaced. The dressing may then be cut to length and placed in a bacteria proof pouch, sealed and sterilised by conventional methods including using ethylene oxide or irradiation.

To make the edge strip cast a solution of polymer onto a strip of film. Screen print the adhesive onto another protector layer and laminate the support layer and polymer to the adhesive in the same manner as above. Remove the casting support film. Trim to the desired width and apply the edge strip at the stage of the preparation of the dressing which involves removing the protector layer, ensuring that the protector layer of the edge strip is removed and replaced by the protector layer of the adhesive dressing.

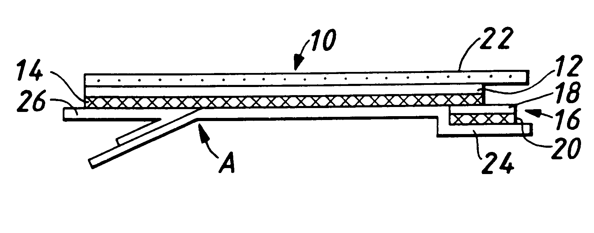

The invention will now be described strictly by way of example, with reference to the accompanying drawing which represents a diagrammatic side view of a dressing according to the invention.

The figure illustrates an adhesive dressing 10 which comprises a backing layer 12 formed from a film of hydrophilic polyurethane. The backing layer 12 has upon one surface thereof a pattern spread pressure sensitive adhesive layer 14 which is formed from an acrylic adhesive. Attached to the non-adhesive surface of the backing layer 12 is a support layer 22 which supports the entire surface of backing layer 12. The support layer 22 extends beyond the backing layer 12. Attached to the adhesive layer 14 along one edge of the dressing 10 is an edge strip generally designated 16. The edge strip 16 has a conformable layer 18 which is adhered to the adhesive layer 14 and has on the remaining surface thereof a layer of skin contacting adhesive 20. The edge strip 16 extends outwardly from the backing layer 12 so as to underlie the support layer 22 which extends beyond the backing layer 12.

The adhesive layer 14 and skin contacting adhesive layer 20 are covered by a removable protector 24 and a smaller removable protector 26 also covers part of the adhesive layer 20. Both of the removable protectors 24 and 26 extend beyond the backing layer 12. The smaller removable protector 26 is folded into a V-shape and the larger removable protector 24 is essentially flat and covers both the adhesive layer 14 and adhesive layer 20 and overlaps on to the smaller cover 26 at a point indicated by the letter A.

In use the larger protector 24 is first removed and the dressing 10 held between an appliers finger and thumb by the removable protector 26 and support layer 22. Once the area of the dressing 10 and edge strip 16 covered by the larger protector 24 is adhered to the skin then the remaining protector 26 may be removed and thus the remaining part of the dressing adhered to the skin of the patient. Immediately following this the support layer 22 is removed by holding onto and peeling back the portion of the support layer 22 which extends beyond the backing layer 12 and which the edge strip 16 underlies.

## Claims

1. An adhesive dressing comprising a conformable backing layer (12) having a pressure sensitive adhesive layer (14) over one surface thereof, a removable protector (24,26) which covers the adhesive layer (14) and extends beyond the backing layer at a contiguous edge on the backing (12) and adhesive layers (14) and a conformable support layer (22) which is releasably attached to the non-adhesive surface of the backing layer (12) and extends beyond the backing layer (12) at said edge characterised in that attached to the adhesive layer (14) along at least a part of said edge is a component (16) comprising a second conformable layer (18) having a skin contacting layer of adhesive (20) on one surface thereof, which component (16) extends outwardly to underlie the extending portion of the conformable support layer (22).

2. An adhesive dressing as claimed in claim 1 wherein the backing layer (12) is vapour permeable.

3. An adhesive dressing as claimed in claim 1 or 2 wherein the backing layer (12) comprises polyester or polyether polyurethanes or polyether polyamides.

4. An adhesive dressing as claimed any preceding wherein the backing layer (12) comprises a hydrophilic polyurethane having a water uptake of 5 to 95% by weight of water.

5. An adhesive dressing as claimed in any preceding claim wherein the adhesive on the backing layer is a moisture vapour permeable adhesive and has a weight of 5gm⁻² to 50 gm⁻².

6. An adhesive dressing as claimed in any preceding claim wherein the layer of adhesive (14) on the backing layer is a discontinuous layer of adhesive spread over the film to allow moisture vapour transmission, by pattern spreading or by spreading a porous adhesive.

7. An adhesive dressing as claimed in any preceding claim wherein the removable protector (24,26) is divided into two or more pieces and at least one of the protector pieces (24) is significantly larger than the other or others (26) and covers a major proportion of the adhesive layer (14) on the backing layer.

8. An adhesive dressing as claimed in any preceding claim wherein the stripping load of the support layer (22) from the backing layer (12) is equal to or greater than that of the protector (24,26) from the adhesive layer (14) on the backing layer.

9. An adhesive dressing as claimed in any preceding claim wherein the removable protector (24,26) covers and extends beyond the second conformable layer (18) and skin contacting adhesive (20) of the component (16) once it has been applied.

10. An adhesive dressing as claimed in any preceding claim wherein the component (16) is a continuous strip attached along the edge of the backing layer.

11. An adhesive dressing as claimed in any of the claims 1-9 wherein the component (16) is discontinuous and attached along only part of the edge of the backing layer.

12. An adhesive dressing as claimed in any of claims 1-9 wherein the component (16) is attached along the edge of all of the backing layer.

13. An adhesive dressing as claimed in any preceding claim wherein the second conformable layer (18) is made from the same material as the backing layer (12) and the skin contacting layer of adhesive (20) is that used upon the backing layer.

14. A method of manufacturing a wound dressing as claimed in claim 1 comprising the steps of:
a) casting a solution of a polymer which is to form a backing layer onto a strip of film forming a support layer
b) spreading an adhesive onto a protector layer and laminating the adhesive and film and those associated layers together,
c) removing the protector layer from the adhesive and, if required, trimming off a strip of film and adhesive along at least one edge of the laminate and characterised in that said method comprises the further steps of applying a component comprising a second conformable layer, adhesive layer and protector to an edge or trimmed edge of the adhesive layer contiguous with the backing layer, removing the protector layer from the component and replacing the protector layer of the adhesive dressing.

## Patentansprüche

1. Haftverband mit einer anpaßbaren Traglage (12) mit einer Selbstkleberschicht (14) auf einer Oberfläche derselben, einer entfernbaren Schutzlage (24, 26), welche die Kleberschicht (14) abdeckt und sich über die Traglage an einer angrenzenden Kante an der Traglage (12) und der Kleberschicht (14) hinaus erstreckt, und einer anpaßbaren Stützlage (22), die lösbar an der nicht-klebenden Oberfläche der Traglage angebracht ist und sich an der Kante über die Traglage (12) hinaus erstreckt, **dadurch gekennzeichnet**, daß an der Kleberschicht (14) entlang wenigstens einem Teil der Kante eine Komponente (16) angebracht ist, die eine zweite anpaßbare Lage (18) mit einer mit der Haut in Berührung kommenden Kleberschicht (20) an einer ihrer Oberflächen aufweist, wobei die Komponente (16) sich nach außen erstreckt und unterhalb des verlängerten Abschnittes der anpaßbaren Stützlage (22) zu liegen kommt.

2. Haftverband nach Anspruch 1, bei welchem die Traglage (12) für Wasserdampf durchlässig ist.

3. Haftverband nach Anspruch 1 oder 2, bei welchem die Traglage (12) Polyester- oder Polyether-Polyurethane oder Polyetherpolyamide umfaßt.

4. Haftverband nach einem der vorhergehenden Ansprüche, bei welchem die Traglage (12) ein hydrophyles Polyurethan mit einer Wasseraufnahme von 5 - 95 Gew.-% Wasser umfaßt.

5. Haftverband nach einem der vorhergehenden Ansprüche, bei welchem der Kleber auf der Traglage ein wasserdampfdurchlässiger Kleber ist mit einem Gewicht von 5 gm⁻² bis 50 gm⁻².

6. Haftverband nach einem der vorhergehenden Ansprüche, bei welchem die Kleberschicht (14) auf der Traglage eine diskontinuierliche Schicht eines auf der Folie aufgetragenen Klebers ist, um eine Wasserdampfdurchlässigkeit durch Auftragen in Form eines Musters oder durch Auftragen eines porösen Klebers zu ermöglichen.

7. Haftverband nach einem der vorhergehenden Ansprüche, bei welchem die entfernbare Schutzlage (24, 26) in zwei oder mehr Stücke unterteilt ist und wenigstens eines der Stücke ((24) der Schutzlage (24) merklich größer ist als das andere oder die anderen (26) und einen größeren Anteil der Kleberschicht (14) auf der Traglage abdeckt.

8. Haftverband nach einem der vorhergehenden Ansprüche, bei welchem die für das Abziehen erforderliche Belastung der Stützlage (22) von der Traglage (12) gleich oder größer ist als die der Schutzlage (24, 26) von der Kleberschicht (14) auf der Traglage.

9. Haftverband nach einem der vorhergehenden Ansprüche, bei welchem die entfernbare Schutzlage (24, 26) die zweite anpaßbare Lage und den mit der Haut in Berührung kommenden Kleber (20) der Komponente (16) abdeckt und sich über diese hinaus erstreckt, nachdem sie aufgebracht worden ist.

10. Haftverband nach einem der vorhergehenden Ansprüche, bei welchem die Komponente (16) ein kontinuierlicher Streifen ist, der entlang der Kante der Traglage angebracht ist.

11. Haftverband nach einem der Ansprüche 1 - 9, bei welchem die Komponente (16) diskontinuierlich und entlang nur einem Teil der Kante der Traglage angebracht ist.

12. Haftverband nach einem der Ansprüche 1 - 9, bei welchem die Komponente (16) entlang der Kante der gesamten Traglage angebracht ist.

13. Haftverband nach einem der vorhergehenden Ansprüche, bei welchem die zweite anpaßbare Lage (18) aus dem gleichen Material hergestellt ist wie die Traglage (12) und die mit der Haut in Berührung kommende Kleberschicht (20) jene ist, die auf der Traglage verwendet wird.

14. Verfahren zum Herstellen eines Wundverbandes gemäß Anspruch 1, der folgende Schritte umfaßt:
a) Gießen einer Lösung eines Polymers, welches eine Traglage bilden soll, auf einem Folienstreifen, der eine Stützlage bildet,
b) Auftragen eines Klebers auf eine Schutzlage und Zusammenlaminieren des Klebers und des Films und der zugehörigen Lagen,
c) Entfernen der Schutzlage vom Kleber und, falls erforderlich, Zurechtschneiden eines Streifens aus Folie und Kleber entlang wenigstens einer Kante des Laminats, **dadurch gekennzeichnet**, daß dieses Verfahren die weiteren Schritte des Aufbringens einer Komponente mit einer zweiten anpaßbaren Lage, einer Klebstoffschicht und einer Schutzlage an einer Kante oder beschnittenen Kante der Kleberschicht, die an die Tragschicht angrenzt, Entfernens der Schutzlage von der Komponente und Wiederanbringens der Schutzlage des Haftverbandes umfaßt.

## Revendications

1. Pansement adhésif comprenant une couche de renforcement (12) épousant les formes dont une face porte une couche d'adhésif (14) sensible à la pression, une protection détachable (24, 26) qui recouvre la couche d'adhésif (14) et se prolonge au-delà de la couche de renforcement au niveau d'un bord contigu sur les couches de renforcement (12) et d'adhésif (14) et une couche de support (22) épousant les formes qui est attachée de façon à pouvoir être détachée à la surface non adhésive de la couche de renforcement (12) et se prolonge au-delà de la couche de renforcement (12) au niveau de ce bord, caractérisé en ce que, attaché à la couche d'adhésif (14) le long d'au moins une partie de ce bord, se trouve un composant (16) comprenant une seconde couche (18) épousant les formes dont une face porte une couche d'adhésif (20) en contact avec la peau, lequel composant (16) se prolonge vers l'extérieur pour être en-dessous de la partie qui se prolonge de la couche de support (22) épousant les formes.

2. Pansement adhésif suivant la revendication 1, dans lequel la couche de renforcement (12) est perméable à la vapeur.

3. Pansement adhésif suivant les revendications 1 ou 2, dans lequel la couche de renforcement (12) comprend des polyester ou polyéther polyuréthanes ou des polyéther polyamides.

4. Pansement adhésif suivant l'une quelconque des revendications précédentes, dans lequel la couche de renforcement (12) comprend un polyuréthane hydrophile ayant une prise d'eau de 5 à 95% en poids d'eau.

5. Pansement adhésif suivant l'une quelconque des revendications précédentes, dans lequel l'adhésif sur la couche de renforcement est un adhésif perméable à la vapeur d'eau et a un poids de 5 g/m² à 50 g/m².

6. Pansement adhésif suivant l'une quelconque des revendications précédentes, dans lequel la couche d'adhésif (14) sur la couche de renforcement est une couche discontinue d'adhésif étalée sur le film pour permettre une transmission de vapeur d'eau, par étalement selon un motif ou par étalement d'un adhésif poreux.

7. Pansement adhésif suivant l'une quelconque des revendications précédentes, dans lequel la protection détachable (24, 26) est divisée en deux morceaux ou plus, et l'un au moins des morceaux de protection (24) est notablement plus grand que l'autre ou les autres morceaux (26) et recouvre une proportion majeure de la couche adhésive (14) sur la couche de renforcement.

8. Pansement adhésif suivant l'une quelconque des revendications précédentes, dans lequel la force pour arracher la couche de support (22) de la couche de renforcement (12) est égale ou supérieure à la force pour arracher la protection (24, 26) de la couche d'adhésif (14) sur la couche de renforcement.

9. Pansement adhésif suivant l'une quelconque des revendications précédentes, dans lequel la protection détachable (24, 26) recouvre la seconde couche (18) épousant les formes et la couche d'adhésif (20) en contact avec la peau du composant (16) et se prolonge au-delà de celles-ci, une fois qu'elle a été appliquée.

10. Pansement adhésif suivant l'une quelconque des revendications précédentes, dans lequel le composant (16) est une bande continue attachée le long du bord de la couche de renforcement.

11. Pansement adhésif suivant l'une quelconque des revendications 1 à 9, dans lequel le composant (16) est discontinu et attaché le long d'une partie seulement du bord de la couche de renforcement.

12. Pansement adhésif suivant l'une quelconque des revendications 1 à 9, dans lequel le composant (16) est attaché le long du bord de toute la couche de renforcement.

13. Pansement adhésif suivant l'une quelconque des revendications précédentes, dans lequel la seconde couche (18) épousant les formes est faite à partir du même matériau que celui qui forme la couche de renforcement (12) et la couche d'adhésif (20) en contact avec la peau est celle qui est utilisée sur la couche de renforcement.

14. Procédé de fabrication d'un pansement pour plaies suivant la revendication 1, comprenant les étapes de :
(a) coulée d'une solution d'un polymère qui doit former une couche de renforcement sur une bande de film formant une couche de support,
(b) étalement d'un adhésif sur une couche de protection et feuilletage de l'adhésif et du film et ces couches associées ensemble,
(c) détachement de la couche de protection de l'adhésif et, si cela est nécessaire, découpe d'une bande de film et d'adhésif le long d'au moins un bord du feuilleté, et caractérisé en ce que ce procédé comprend les étapes supplémentaires d'application d'un composant comprenant une seconde couche épousant les formes, une couche adhésive et une protection sur un bord ou un bord découpé de la couche d'adhésif contigu à la couche de renforcement, enlèvement de la couche de protection du composant et remise en place de la couche de protection du pansement adhésif.
